# EUROPEAN PATENT APPLICATION

(11) **EP 2 305 284 A2**
(43) Date of publication of application: **06.04.2011**
(21) Application number: 10182004.1
(22) Date of filing: 07.03.2003
(51) Int. Cl.: A61K 38/16, A61K 38/18, A61K 38/48, C07K 16/28, A61K 31/381, A61K 31/16

(54) **Use of EGFR transactivation inhibitors in human cancer**

(30) Priority: 08.03.2002 EP 02005452
(62) Divisional of application: 03714791.5
(71) Applicant: Max-Planck-Gesellschaft zur Förderung der Wissenschaften e.V., 80539 München (DE)
(72) Inventor: Ullrich, Axel, 80331 München (DE); Förster, Beatrix, 81241 München (DE); Fischer, Oliver, Canterbury Kent CT2 8AN (GB); Gschwind, Andreas, 1130 Wien (AT); Leserer, Michael, 82110 Germering (DE)
(74) Representative: Weiss, Wolfgang

(57) **Abstract**

The present invention relates to the use of a compound which is capable of inhibiting activation of a growth factor receptor of the EGFR-family for the prevention or treatment of processes associated with increased G-protein mediated signal transduction.

## Description

The present invention relates to the use of a compound which is capable of inhibiting activation of growth-factor receptors of the EGFR-family for the prevention or treatment of processes associated with increased G-protein mediated signal transduction.

Signaling through receptor tyrosine kinases (RTK) is involved in the regulation of fundamentally important cellular processes and its upregulation has been shown to be connected to hyperproliferative diseases such as cancer. G protein-coupled-receptors (GPCR) constitute the largest group of cell surface receptors controlling multiple signaling cascades and their biological outcome. Recently, crosstalk between members of both receptor families has been described that connects the multitude of different stimuli via GPCR ligands with the signaling capability of RTKs such as the epidermal growth factor receptor (EGFR), see e.g. WO01/12182. The signaling mechanism which involves shedding of growth-factor precursors by a metalloprotease led us to propose the model of the triple-membrane-passing-signal (TMPS) pathway.

Our object was to investigate the physiological processes that are regulated by the TMPS pathway and its significance for pathophysiological phenomena such as neoplastic progression. Therefore, we screened human cancer cell lines for EGFR transactivation by stimulation with GPCR ligands, as well as inhibition of metalloproteases. Specifically, we investigated growth factor-stimulated events in signal transduction and in defined physiological processes.

Our results show that EGFR phosphorylation as well as downstream signaling events such as the recruitment of adapter proteins and phosphorylation of the mitogen-activated protein kinase occur after stimulation with GPCR ligands and are downregulated by the metalloprotease inhibitor batimastat. Additionally, basal phosphorylation of the EGFR is batimastat-sensitive. Furthermore, we revealed that EGFR transactivation is part of the regulatory system which modulates cell cycle progression and cell proliferation. The TMPS pathway is also able to promote anti-apoptosis, cell migration and invasivity.

While somatic cells require external mitogenic signals, cancer cells are characterized by abnormal growth behaviour due to autocrine production of mitogenic factors. As many of these are GPCR ligands, EGFR transactivation constitutes a mechanism for cancer progression by deregulation of cell proliferation and suppression of cell death. Inhibition of this pathway is therefore a promising strategy for the treatment of cancer.

Thus, a subject matter of the present invention is the use of a compound which is capable of inhibiting activation of a growth-factor receptor of the EGFR family for the manufacture of an agent for the prevention or treatment of processes selected from cell proliferation, cell migration, invasivity and anti-apoptosis in a disorder, which is associated with increased G-protein mediated signal transduction.

Surprisingly, it was found that inhibition of growth-factor receptor activation caused by increased G-protein mediated signal transduction leads to an inhibition of cancer progression, particularly cell migration and invasivity, as well as to an inhibition of anti-apoptosis. Thus, inhibitors of the GPCR-induced growth-factor receptor activation are suitable for the manufacture of medicaments for the prevention or treatment of specific indication of hyper-proliferative diseases associated with cell-proliferation, cell migration, invasivity and/or anti-apoptosis and to re-establish control of these phenomena in the treated cells or organisms, respectively.

The growth-factor receptor is preferably EGFR or another member of the EGFR family, such as HER-2, HER-3 or HER-4, but also other growth-factor receptors, particularly receptor tyrosine-kinases may be inhibited.

The compound may act on a growth-factor receptor ligand precursor, which is preferably a membrane-associated molecule. In a particularly preferred embodiment the growth-factor ligand precursor is pro-HB-EGF which is cleaved to HB-EGF by a protease. Other preferred examples of growth-factor receptor ligands which are cleaved from precursors are other members of the EGF family, such as TGF-α, amphiregulin, epi-regulin, EGF, β-cellulin and members of the heregulin/NDF family including isoforms thereof.

An example of a compound, which acts on a growth-factor receptor ligand precursor, is CRM197, a catalytically inactive form of the diphteria toxin, which specifically binds to pro-HB-EGF and which is capable of blocking the processing of pro-HB-EGF. A further example is an antibody, which is capable of binding to pro-HB-EGF and which thereby blocks its processing.

In a further embodiment the compound acts on a metalloprotease, particularly a membrane-associated metalloprotease, e.g. a protease of the ADAM family. Inhibition of the protease leads to a blocking of the processing of growth-factor receptor ligand precursors and thus results in an inhibition of growth-factor receptor activation. Preferred examples of inhibitors of protease activity are batimastat (BB-94), marimastat, TAPI and TIMP-1-2-3 or -4, particularly TIMP-3.

In a still further embodiment the compound may act on the growth-factor receptor itself. For example, the compound may bind to the growth-factor receptor and thereby inhibit transactivation. An example of such a compound is the EGFR-inhibitor AG1478. Further, growth-factor receptor-binding antibodies may be used.

It should be noted that the present invention relates to a targeted inhibition of cellular signal pathways "downstream" of the EGFR transactivation in cancer cells, particularly in human cancer cells.

The disorder to be treated or prevented is associated with and preferably caused by increased G protein-mediated signal transduction. This increased G protein-mediated signal transduction may be associated with or caused by a pathological increase in the activity of a G protein and/or a G protein-coupled receptor (GPCR). It should be noted that the disorders which are prevented or treated according to the present invention can be delimited from other hyperproliferative disorders having an enhanced growth-factor receptor expression in that a transactivation of growth-factor receptor expression via G protein signal pathways takes place. More particularly, the disorder is a cancer, such as a colon, kidney, liver, bladder, pancreatic, prostate, gastric, breast, lung, thyroid, pitnitary, adrenal or ovarian tumor or glioblastoma.

Pharmaceutical compositions suitable for use in the present invention include compositions wherein the active ingredients are contained in an effective amount to achieve its intended purpose. A therapeutically effective dose refers to that amount of the compound that results in amelioration of symptoms or a prolongation of survival in a patient. Toxicity and therapeutic efficacy of such compounds can be determined by standard pharmaceutical procedures in cell cultures or experimental animals, e.g. for determining the LD50 (the dose lethal to 50% of the population) and the ED50 (the dose therapeutically effective in 50% of the population). For any compound used in the method of the invention, the therapeutically effective dose can be estimated initially from cell culture assays. For example, a dose can be formulated in animal models to achieve a circulating concentration range that includes the IC50 as determined in cell culture (i.e. the concentration of the test compound which achieves a half-maximal inhibition of the growth-factor receptor activity). Such information can be used to more accurately determine useful doses in humans. The dose ratio between toxic and therapeutic effects is the therapeutic index and it can be expressed as the ratio between LD50 and ED50. Compounds which exhibit high therapeutic indices are preferred. The exact formulation, route of administration and dosage can be chosen by the individual physician in view of the patient's condition (see e.g. Fingl et al., 1975, in "The Pharmacological Basis of Therapeutics", Ch. 1, p. 1). Dosage amount and interval may be adjusted individually to provide plasma levels of the active moiety which are sufficient to maintain the receptor modulating effects, or minimal effective concentration (MEC). The MEC will vary for each compound but can be estimated from in vitro data, e.g. the concentration necessary to achieve a 50-90% inhibition of the receptor using the assays described herein. Compounds should be administered using a regimen which maintains plasma levels above the MEC for 10-90% of the time, preferably between 30-90% and most preferably between 50-90%. Dosages necessary to achieve the MEC will depend on individual characteristics and route of administration. In cases of local administration or selective uptake, the effective local concentration of the drug may not be related to plasma concentration.

The actual amount of composition administered will, of course, be dependent on the subject being treated, on the subject's weight, the severity of the affliction, the manner of administration and the judgement of the prescribing physician. For batimastat, and other compounds e.g. a daily dosage of 1 to 200 mg/kg, particularly 10 to 100 mg/kg per day is suitable.

Further, the invention relates to a method for identifying and/or characterizing an inhibitor of processes selected from cell proliferation, cell migration, invasivity and anti-apoptosis in a disorder associated with increased G-protein mediated signal transduction, comprising: determining the effect of a test compound on the transactivation of a growth-factor receptor of the EGFR familiy. This method is proferably a screening method which comprises a functional assay for cell proliferation, cell migration, invasivity and/or anti-apoptosis, e.g. as described in the examples. The test compounds may be selected from low-molecular weight compounds, peptides and proteins, particularly antibodies or antibody fragments.

Further, the invention shall be explained in more detail by the following examples:

### EXAMPLE

### 1. METHODS

### 1.1 Cell lysis, immunoprecipitation and immunoblotting

Prior to lysis, cells grown to 80% confluency were treated with inhibitors and agonists and lysed for 10 min on ice in buffer containing 50 mM HEPES pH 7.5, 150 mM NaCl, 1% Triton X-100, 1 mM EDTA, 10% glycerol, 10 mM sodium pyrophosphate, 2mM sodium orthovanadate, 10 mM sodium fluoride, 1 mM phenylmethylsulfonyl fluoride and 10 µg/ml aprotinin. Lysates were precleared by centrifugation at 13000 rpm for 10 min at 4°C. Supernatants were diluted with an equal volume of HNTG buffer and subsequently immunoprecipitated using the respective antibodies and 30 µl of protein A-Sepharose for 4 h at 4°C. Precipitates were washed three times with 0.5 ml of HNTG buffer, suspended in SDS sample buffer and subjected on gel electrophoresis on 7.5% or 10% gels. Following SDS page, proteins were transferred to a nitrocellulose membrane and immunoblotted.

### 1.2 Invasion assay

Cell invasion assays were performed in Boyden chambers. Serumfree medium containing the chemoattractant to be tested is added to the lower well of a Boyden chamber. A Matrigel coated filter is placed over the lower well of the Boyden chamber and is secured with a gasket. The cells are preincubated with the inhibitor for 20 min and then added to the upper wells of the chambers in serumfree medium. The chambers are incubated for 6 h to overnight in a humified 7% CO₂, 37°C incubator. Finally, cells are wiped from the upper surface with a cotton tip swab and the cells on the lower side are stained and counted under the microscope.

### 1.3 Migration assay

Cell migration assays were also performed in Boyden chambers containing polycarbonate membranes (8 µm pore size) as described above.

### 1.4 Cell wounding assay

Cells were grown to confluence in six well plates in standard medium and analysed using a classical scratch wound method. Cells were gently scraped with a plastic tip. The medium was removed, and cells were washed twice with PBS. Serumfree Medium was added and the cells were permitted to migrate into the area of clearing for 24 h to 48 h. Analysis was performed under the microscope.

### 1.5 Thymidine incorporation

Cells were seeded in 12 well plates grown to 70% confluence, and then incubated in serum free medium for 24 h to induce quiescence. Mitogenic stimuli were then added at time 0, when required pre-treatment with inhibitors. After 18 h 1 µ Cl (methyl-³H) thymidine was added to each well. Four hours later the culture media was removed, cells were washed with PBS, fixed with ice could 10% trichloroacetic acid. Labelled material was then solubilized and cell associated radioactivity was then quantified by liquid scintillation counting.

### 1.6 Detection of Apoptosis

Apoptosis was measured by flow cytometry. Cells were seeded in six well plates and starved. Apoptosis was induced by anti-CD95 antibody, at the same time inhibitors and afterwards mitogens were added after 24 h. At the end of the culture period cells were washed in PBS, resuspended in hypotonic buffer containing 50 µg/ml propidium iodide, kept for 2 h at 4°C in the dark, and analysed by flow cytometry. The percentage of apoptotic cells was determined by evaluating hypodiploid nuclei after proper gating on DNA content.

### 2. RESULTS

Investigation of the physiological processes that are regulated by EGFR transactivation via the triple-membrane-passing signal (TMPS) pathway:
1. Several human cancer cell lines show EGFR transactivation by GPCR ligands as well as inhibition of metalloproteases.
2. Our results show that EGFR phosphorylation as well as downstream signaling events such as the recruitment of adapter proteins and phosphorylation of the mitogen activated protein kinase occur after stimulation with GPCR ligands and are downregulated by the metalloprotease inhibitor batimastat. Additionally, basal phosphorylation of the EGFR is batimastat sensitive.
3. EGFR transactivation via the TMPS pathway promotes cell proliferation, anti-apoptosis, cell migration and invasion.

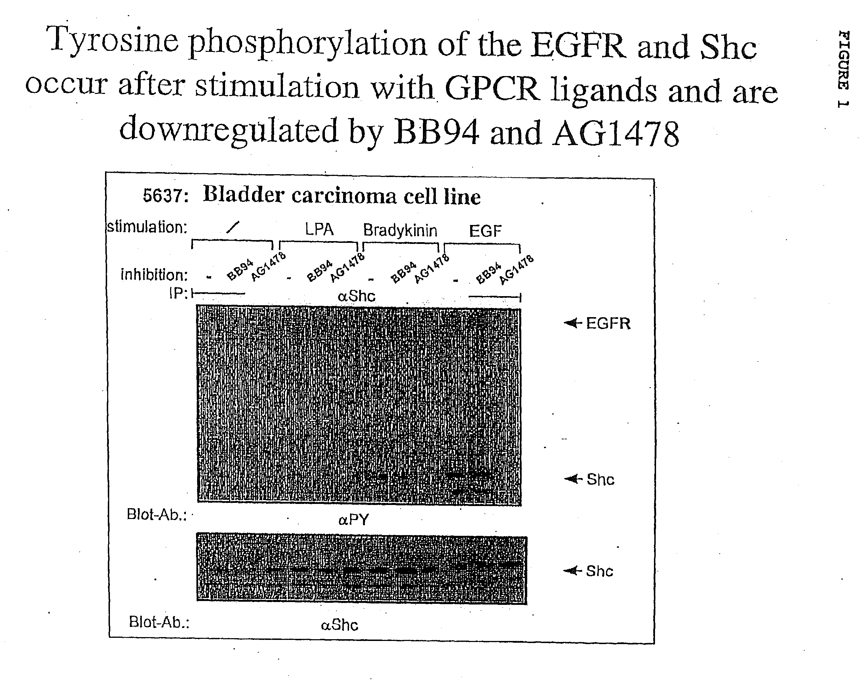

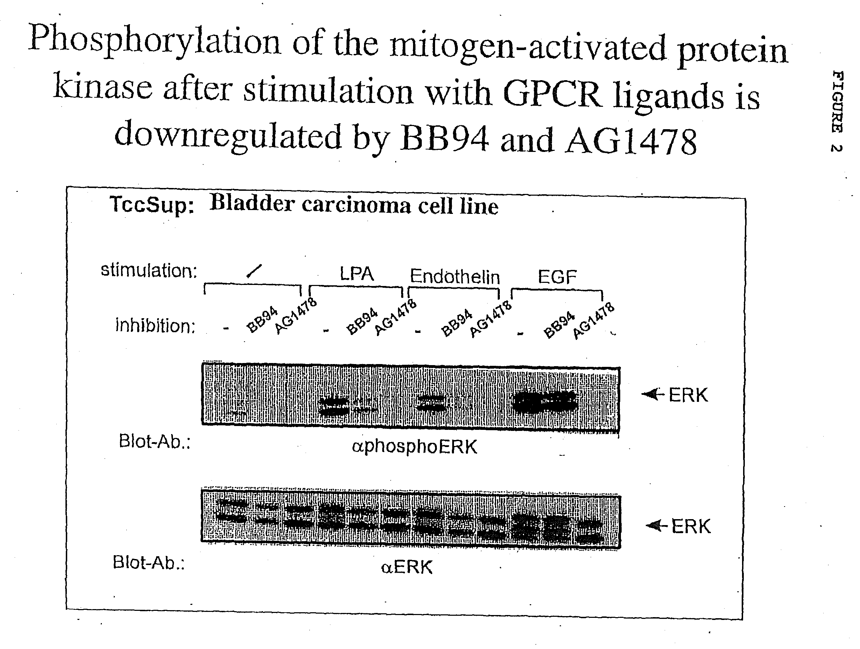

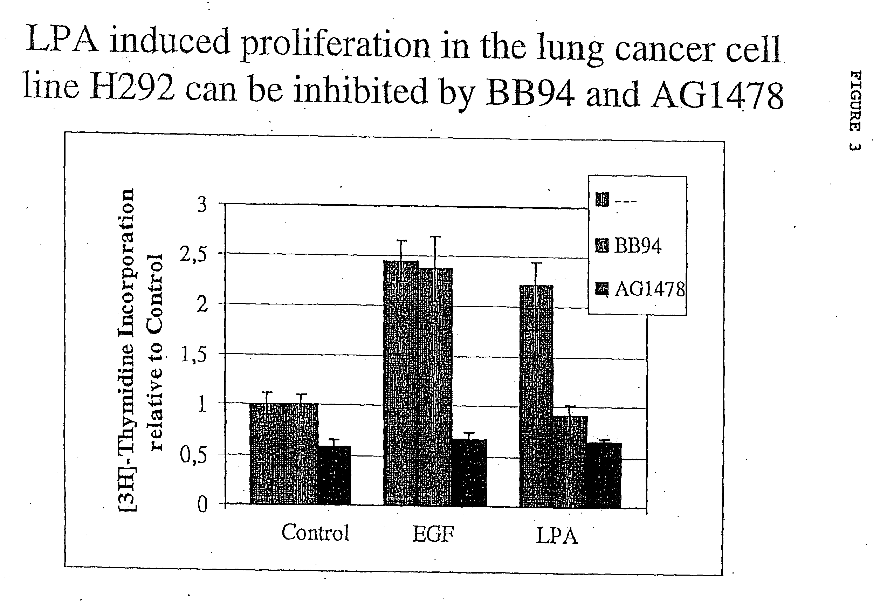

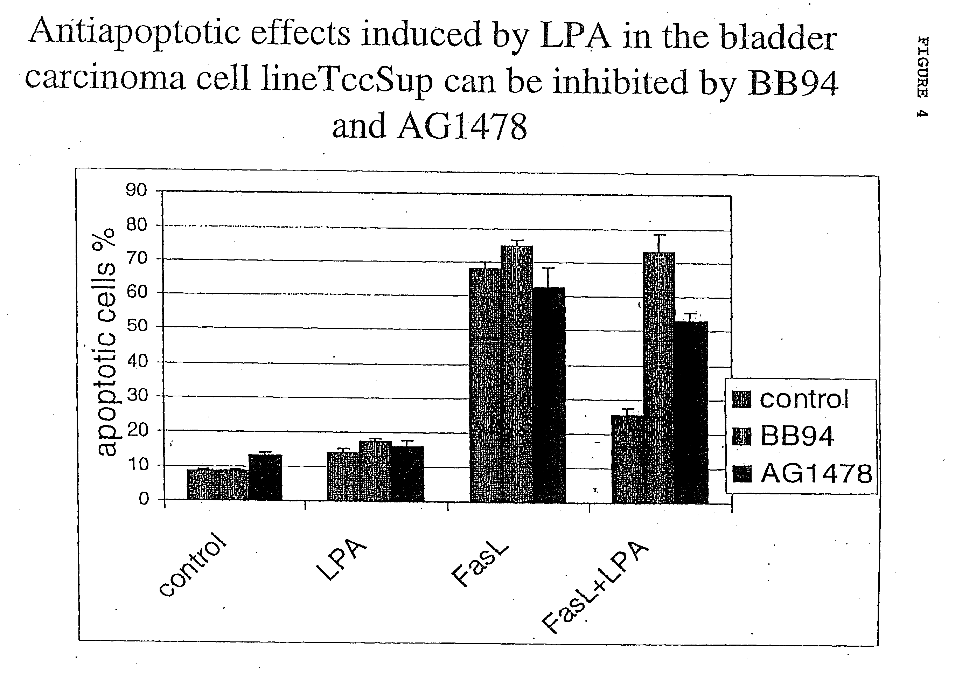

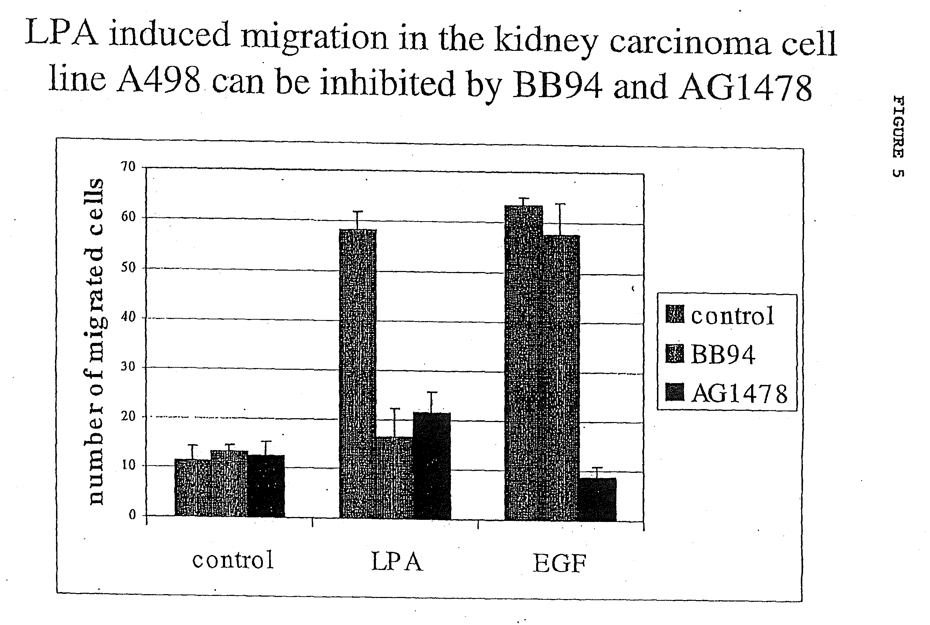

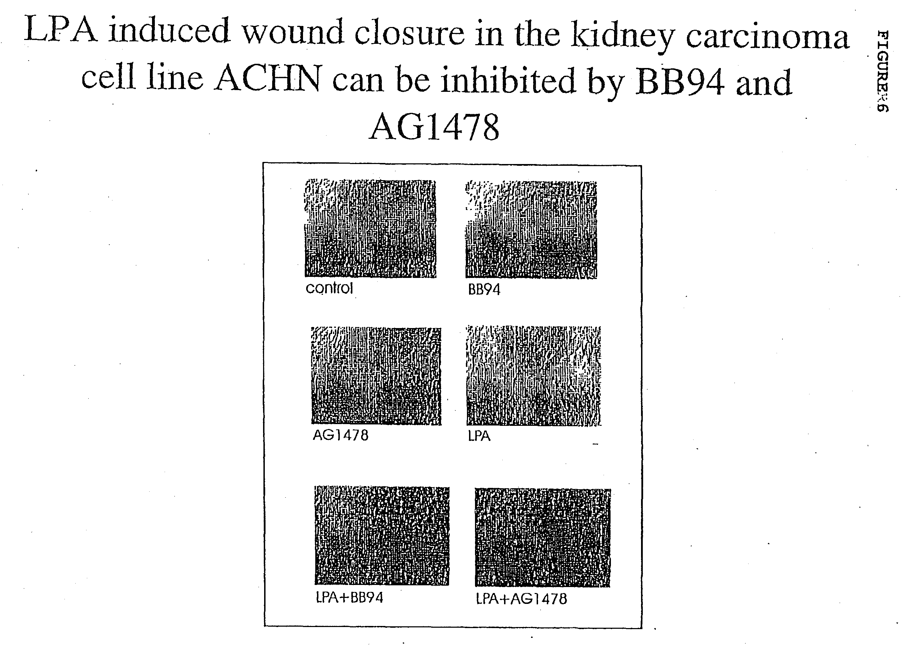

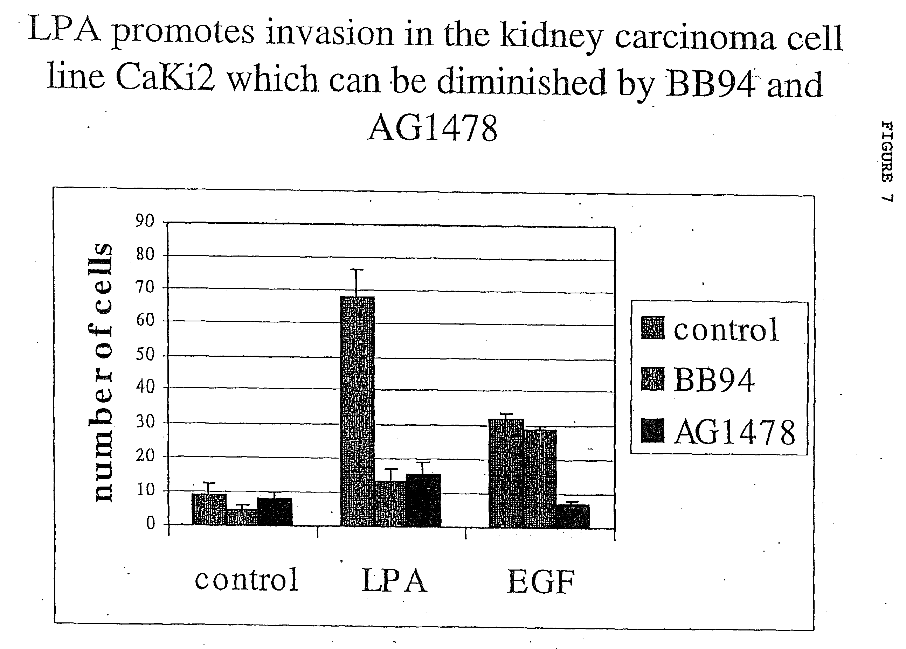

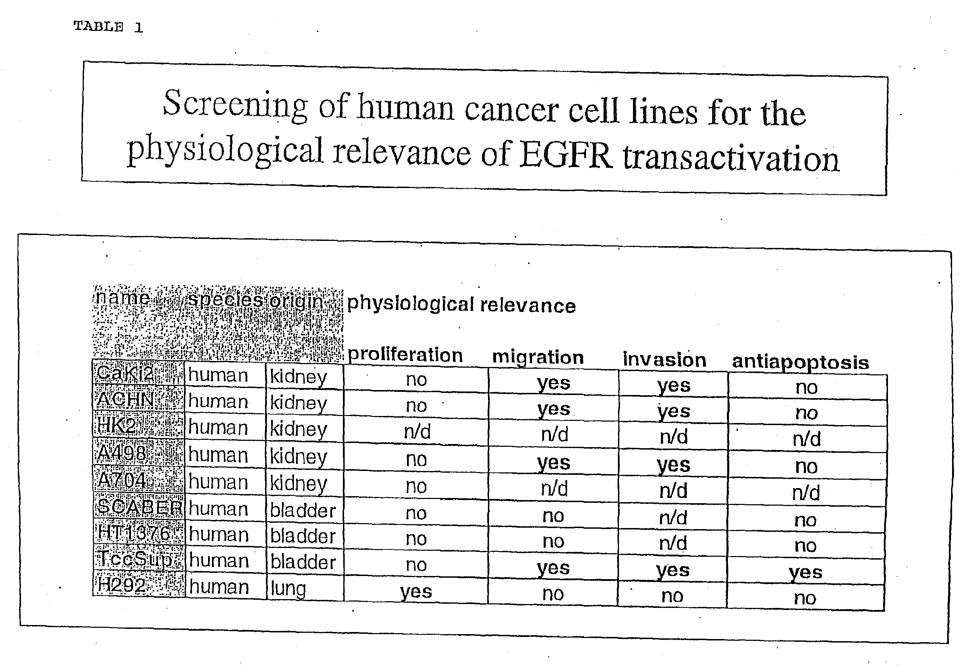

The results are summarized in Fig. 1-7 and in Table 1.

### 3. CONCLUSIONS

Cancer cells are characterized by abnormal growth behaviour due to autocrine production of mitogenic factors. As many of these are GPCR ligands, EGFR transactivation constitutes a likely mechanism for cancer progression by deregulation of cell proliferation and suppression of cell death. Inhibition of this pathway is therefore a promising strategy for the treatment of cancer.

## Claims

1. Use of a compound which is capable of inhibiting activation of a growth-factor receptor of the EGFR family for the manufacture of an agent for the prevention or treatment of processes selected from cell proliferation, cell migration, invasivity and anti-apoptosis in a disorder, which is associated with increased G-protein mediated signal transduction.

2. The use of claim 1 wherein the growth-factor receptor is EGFR.

3. The use of claim 1 or 2 wherein the compound acts on a growth-factor receptor ligand precursor.

4. The use of claim 3 wherein the growth-factor receptor ligand precursor is EGF or an EGF-like ligand.

5. The use of claim 1 or 2 wherein the compound acts on a metalloprotease.

6. The use of claim 5 wherein the compound directly inhibits the protease activity.

7. The use of claim 1 or 2 wherein the compound acts on the growth-factor receptor.

8. The use of any one of claims 1 to 7 wherein the agent is a pharmaceutical composition comprising at least one pharmaceutically acceptable carrier, diluent and/or adjuvant.

9. The use of any one of claims 1 to 8 wherein the disorder is cancer.

10. The use of any one of claims 1 to 9 wherein the cancer is a human cancer.

11. A method for identifying and/or characterizing an inhibitor of processes selected from cell proliferation, cell migration, invasivity and anti-apoptosis in a disorder associated with increased G-protein mediated signal transduction, comprising:
determining the effect of a test compound on the transactivation of a growth-factor receptor of the EGFR familiy.

12. The method of disclaim 11, wherein the test compound is selected from low-molecular weight compounds, peptides and proteins, particularly antibodies or antibody fragments.
